# EUROPEAN PATENT APPLICATION

(11) **EP 1 008 356 A1**
(43) Date of publication of application: **14.06.2000**
(21) Application number: 98905737.7
(22) Date of filing: 03.03.1998
(51) Int. Cl.: A61K 49/04

(54) **X-RAY CONTRAST MEDIA**

(30) Priority: 04.03.1997 JP 4889797
(71) Applicant: Kowa Co., Ltd., Naka-ku, Nagoya-shi, Aichi 460-0003 (JP)
(72) Inventor: INAGI, Toshio, Mishima-shi, Shizuoka 411-0038 (JP); SHINKAWA, Yasuhiro, Fujishi, Shizuoka 417-0809 (JP); OKANO, Teruo, Ichikawashi, Chiba 272-0827 (JP)
(74) Representative: Hartz, Nikolai F., Dr.
(86) International application number: JP9800868
(87) International publication number: WO9839036

(57) **Abstract**

The invention relates to an X-ray contrast medium comprising an X-ray contrast substance coated with a polymer which adheres to the surface of a gastrointestinal mucosa under acid conditions and separates from the surface of the gastrointestinal mucosa under neutral or alkaline conditions. The X-ray contrast medium has excellent adhesiveness to a digestive tract, so that its doses and concentration can be reduced.

## Description

### TECHNICAL FIELD

The present invention relates to an X-ray contrast medium having pH-dependent adherability to the surface of a gastrointestinal mucosa.

### BACKGROUND ART

A contrast radiography for the digestive tract is a method comprising administering a contrast medium having a nature to absorb X-rays to the digestive tract of a subject and taking a radiograph of the inner surface of the digestive tract making good use of a difference in X-ray adsorption, and is used as a method for diagnosing and examining a disease of the digestive tract, such as cancer or tumor (see Heizaburo Ichikawa and Yuji Yoshida, "How to Consider and Forward X-ray Diagnosis of Stomach", Igaku Shoin).

In this method, barium sulfate which is safe and cheap is generally used as an X-ray contrast medium.

In order to take a radiograph of the details of the surface of the gastrointestinal mucosa by the contrast radiography, barium sulfate as a contrast medium must be caused to thinly and evenly adhere to the surface of the gastrointestinal mucosa. However, barium sulfate has a drawback that its adhesiveness to the gastrointestinal mucosa is weak.

On the other hand, it has been reported that a thickening agent, suspending agent or the like is added to barium sulfate to improve the adhesiveness thereof (Japanese Patent Application Laid-Open Nos. 54-160748 and 55-127322).

However, the addition of the thickening agent involves such a problem that the viscosity of the contrast medium is increased to cause uneven adhesion of barium sulfate, and so irregularities due to sticking or uneven adhesion occur on the resulting radiograph.

Under the circumstances, it has accordingly been conducted to administer high-concentration barium sulfate in a great amount (generally about 300 cc) for the purpose of enhancing the adhesiveness. However, such a method has involved problems such as difficulties in administering and excreting the contrast medium, and bellyache upon the excretion. There has hence been a demand for solution of such problems.

It is accordingly an object of the present invention to provide an X-ray contrast medium which has excellent adhesiveness to the digestive tract and is easy to administer and excrete because of its low concentration and small amount.

### DISCLOSURE OF THE INVENTION

In view of the foregoing circumstances, the present inventor has carried out an extensive investigation. As a result, it has been found, from the finding that sugar chains are present on a gastrointestinal mucosa and cell membranes of the mucosa and have hydrogen-bonding ability under acid conditions (see Kalpana R. Kamath and Kinam Park, "Mucosal Adhesive Preparations", Encyclopedia of Pharmaceutical Technology, Volume 10), that when a polymer, which adheres by hydrogen bonding to the digestive tract under the acid conditions and separates from the digestive tract under neutral or alkaline conditions, is coated on an X-ray contrast substance such as barium sulfate, the contrast substance can be caused to thinly and evenly adhere to the digestive tract upon an examination, thereby achieving an excellent contrast effect, and is separated from the digestive tract by increasing the pH within the digestive tract after the examination, thus leading to completion of the present invention.

According to the present invention, there is thus provided an X-ray contrast medium comprising an X-ray contrast substance coated with a polymer which adheres to the surface of a gastrointestinal mucosa under acid conditions and separates from the surface of the gastrointestinal mucosa under neutral or alkaline conditions.

According to the present invention, there is also provided an X-ray contrast composition comprising an X-ray contrast substance coated with a polymer which adheres to the surface of a gastrointestinal mucosa under acid conditions and separates from the surface of the gastrointestinal mucosa under neutral or alkaline conditions, and a pharmaceutically acceptable carrier.

According to the present invention, there is further provided use of an X-ray contrast substance coated with a polymer, which adheres to the surface of a gastrointestinal mucosa under acid conditions and separates from the surface of the gastrointestinal mucosa under neutral or alkaline conditions, for an X-ray contrast medium.

According to the present invention, there is still further provided a contrast-radiographing process, comprising the steps of administering an X-ray contrast substance coated with a polymer, which adheres to the surface of a gastrointestinal mucosa under acid conditions and separates from the surface of the gastrointestinal mucosa under neutral or alkaline conditions, to a subject and exposing the subject to X-rays.

### BRIEF DESCRIPTION OF THE DRAWINGS

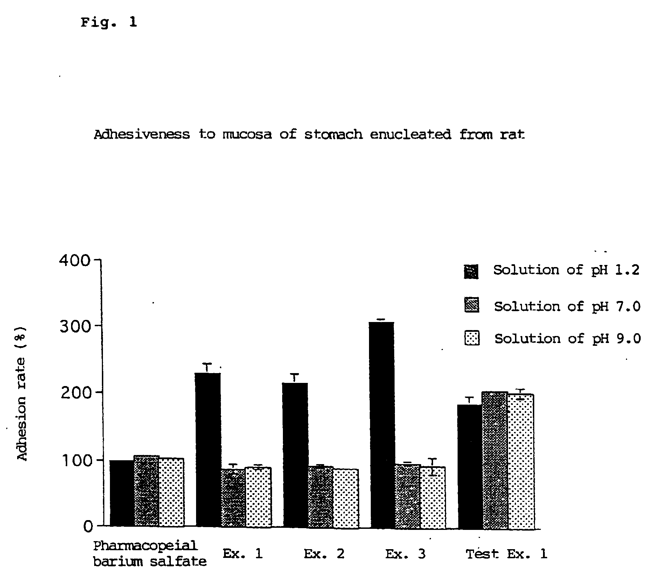

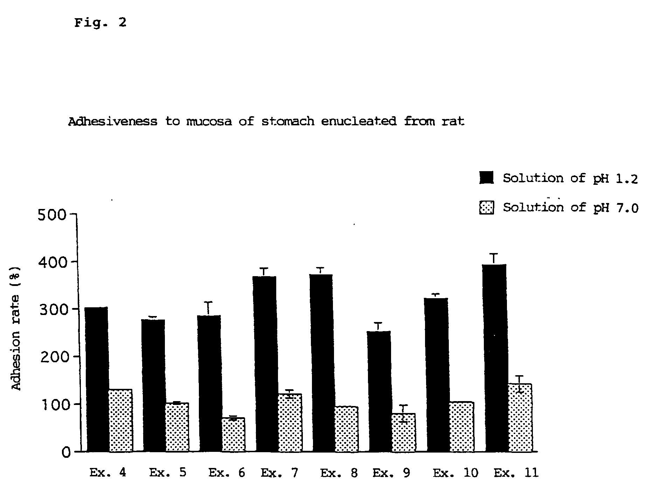

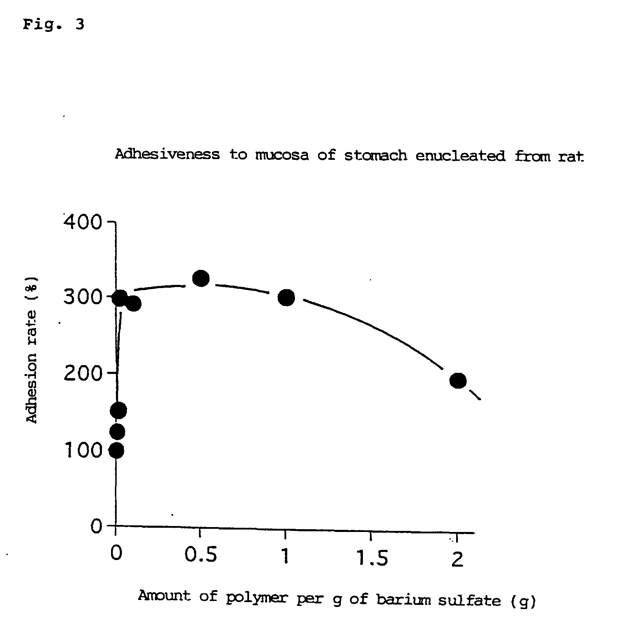

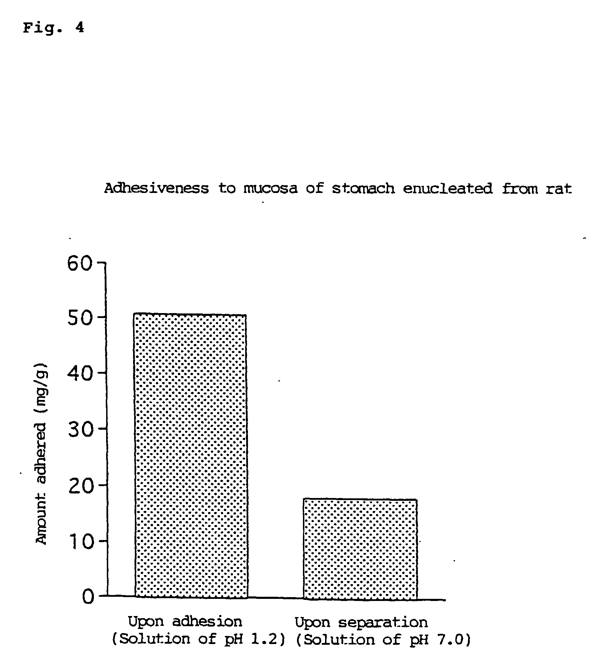

FIG. 1 diagrammatically illustrates the adhesiveness of various contrast media to a mucosa of the stomach enucleated from a rat. FIG. 2 diagrammatically illustrates the adhesiveness of contrast media according to the present invention to a mucosa of the stomach enucleated from a rat. FIG. 3 diagrammatically illustrates the adhesiveness of contrast media, in which the amount of a polymer is varied, to a mucosa of the stomach enucleated from a rat. FIG. 4 diagrammatically illustrates the adhesiveness of a contrast medium to a mucosa of the stomach enucleated from a rat at a varied pH.

### BEST MODE FOR CARRYING OUT THE INVENTION

No particular limitation is imposed on the X-ray contrast substance useful in the practice of the present invention so far as it has a nature to absorb X-rays and is a pharmaceutically acceptable substance. However, examples thereof include the following substances:
(1) Alkaline earth metal compounds:
   barium sulfate, barium chloride, barium hydroxide, barium carbonate, calcium hydroxide, calcium carbonate, calcium phosphate, magnesium hydroxide, magnesium oxide, magnesium carbonate, etc.;
(2) Iron compounds:
   iron chlorides, iron oxides, ferrothreonine, etc.;
(3) Bismuth compounds: bismuth oxycarbonate, etc.; and
(4) X-ray contrast substances of the organic iodine type:
   amidotrizoic acid, ioxaglic acid, iotalamic acid, iotroxatic acid, iotrolan, iopanoic acid, iopamidol, iohexol, sodium iopodate, iodamide, iodoxamic acid, etc.

Of these, barium sulfate is preferred in the present invention.

No particular limitation is imposed on the polymer to be coated on the X-ray contrast substance so far as it adheres to the surface of a gastrointestinal mucosa under acid conditions and separates from the surface thereof under neutral or alkaline conditions. However, it is preferably a polymer exhibiting a pH of 4 or higher when dissolved, and having an anionic group. Examples of such a polymer include the following polymers:
(1) Natural polymers: purified shellac and white shellac; and
(2) Synthetic polymers:
   Cellulose derivative polymers: hydroxypropylmethylcellulose phthalate, hydroxypropylmethylcellulose acetate succinate, carboxymethylethyl cellulose, cellulose acetate trimellitate, cellulose acetate phthalate, etc.;
Acrylic polymers:
   polymers obtained from acrylic acid and/or methacrylic acid, polymers obtained from acrylic acid and/or methacrylic acid and a carboxylic ester, etc.; and
Polyvinyl alcohol type polymers:
   polyvinyl acetate phthalate, etc.

The polymer used in the present invention particularly preferably has a carboxyl group. Such a polymer is preferably that obtained from acrylic acid and/or methacrylic acid, and more preferably from acrylic acid and/or methacrylic acid and a carboxylic ester. Carboxylic ester used herein include acrylic esters and methacrylic esters, for example, methyl acrylate, ethyl acrylate, n-propyl acrylate, isopropyl acrylate, n-butyl acrylate, isobutyl acrylate, t-butyl acrylate, 2-hydroxyethyl acrylate, 2-hydroxypropyl acrylate, methyl methacrylate, ethyl methacrylate, n-propyl methacrylate, isopropyl methacrylate, 2-hydroxyethyl methacrylate, 2-hydroxypropyl methacrylate, n-butyl methacrylate, isobutyl methacrylate, t-butyl methacrylate and the like.

A particularly preferred polymer used in the present invention is a methacrylic acid-methyl methacrylate copolymer (trade name: Eudragit L100).

When the polymer used in the present invention is prepared, a binder, crosslinking agent, polymerization initiator, polymerization promotor, plasticizer and the like may be incorporated in addition to the monomer component. Examples of the binder used herein include alginic acid and salts thereof , pectin, tragacanth, xanthan gum, carboxymethyl cellulose and salts thereof, polyvinyl alcohol, and polyvinyl pyrrolidone. Examples of the crosslinking agent include ethylene glycol di(meth)acrylate, diethylene glycol di(meth)acrylate, triethylene glycol di(meth)acrylate, polyethylene glycol di(meth)acrylate and N,N' -methylenebisacrylamide.

Examples of the polymerization initiator include ammonium peroxodisulfate, benzoyl peroxide, 2,2'-azobis-isobutyronitrile and 2,2' -azobis(2-methylbutyronitrile). An example of the polymerization promotor includes N,N,N',N' -tetramethylethylenediamine. Examples of the plasticizer include triethyl citrate, polyethylene glycol, triacetin, castor oil, glycerol and polysorbate 80.

Processes for coating the X-ray contrast substance with the polymer in the preparation of the X-ray contrast medium according to the present invention include a process in which the X-ray contrast substance is added to a raw stock (i.e., monomer component, etc.) for the polymer to polymerize the monomer component by a suspension polymerization process or seed polymerization process, and the resultant mixture is ground; and a process in which a raw stock for the polymer is coating on the X-ray contrast substance by a spray drying process or fluidized bed coating process.

The coating weight of the polymer in the present invention varies according to the kind of the X-ray contrast substance used. However, it is preferable to apply the polymer in a proportion of generally about 0.005-2 g, particularly about 0.02-1 g per g of the X-ray contrast substance.

In addition to the above components, additives such as a thickening agent, suspending agent, sweetening agent, flavoring agent, antiseptic, anti-foaming agent and water may be added to the X-ray contrast medium according to the present invention as needed.

No particular limitation is imposed on the preparation form of the X-ray contrast medium according to the present invention so far as it may be for oral administration. Specific examples thereof include powder, granules, tablets and suspension. Of these, powder and suspension are preferred because of their easy administration and high contact frequency with the surface of a gastrointestinal mucosa. The preparations of these forms can be formulated in accordance with methods known *per se* in the art.

The dose of the X-ray contrast medium according to the present invention may be suitably determined by species, sex and age. In the case of the human, it is preferable to administer the contrast medium in a dose of generally 10-300 ml per time. However, the X-ray contrast medium according to the present invention can achieve the equivalent contrast effect in an amount a half to a quarter of the conventional contrast medium composed of only barium sulfate. Therefore, the dose may also be reduced to about a half to a quarter.

The X-ray contrast medium according to the present invention is useful for contrast radiography of a gastrointestinal mucosa, further a proximal gastrointestinal mucosa, particularly an esophageal, gastric mucosa and/or duodenal mucosa.

### EXAMPLES

The present invention will hereinafter be described in more detail by the following examples.

### Examples 1-3:

Pharmacopeial barium sulfate was suspended in solutions containing a monomer component, ethylene glycol dimethacrylate (EGDMA) as a crosslinking agent and 2,2'-azobis(2-methylbutyronitrile) (V-59) as a polymerization initiator according to their corresponding formulations shown in Table 1, to polymerize the respective monomer components at 40°C for 48 hours, and the resultant products were then ground, thereby obtaining polymer-coated barium sulfate preparations in the form of powder.

### Comparative Example 1:

A barium sulfate preparation coated with a polymer having no carboxyl group was obtained in the same manner as in Examples 1-3 except that components of its corresponding formulation shown in Table 1 were used.

**Table 1**

| Component | Example 1 | Example 2 | Example 3 | Comparative Example 1 |
|---|---|---|---|---|
| Acrylic acid | 17.27 g | - | 8.65 g | - |
| Methacrylic acid | - | 18.50 g | 10.33 g | - |
| n-Butyl methacrylate | - | - | 6.73 g | 21.01 g |
| EGDMA | 0.48 g | 0.48 g | 0.48 g | 0.48 g |
| V-59 | 0.23g | 0.23 g | 0.23 g | 0.23 g |
| Pharmacopeial barium sulfate | 17.98 g | 19.21 g | 26.42 g | 21.72 g |

### Test Example 1:

With respect to the respective preparations of Examples 1-3 and Comparative Example 1 and pharmacopeial barium sulfate, the adhesiveness to the surface of a mucosa of the stomach enucleated from a rat was investigated. More specifically, each (0.5 g) of the polymer-coated barium sulfate preparations and the pharmacopeial barium sulfate was suspended in each (19.5 g) of aqueous solutions of pH 1.2 (sodium chloride + hydrochloric acid), pH 7.0 (potassium dihydrogenphosphate + sodium hydroxide) and pH 9.0 (boric acid + potassium chloride + sodium hydroxide). The stomach enucleated from the rat was placed into the suspension with the surface of the gastric mucosa outside. After the suspension was stirred for 10 minutes, the stomach was taken out of the suspension, thereby determining the amount of the preparation adhered to the surface of the gastric mucosa by measuring the dry weight of the preparation remaining in the suspension of each pH.

The results are illustrated in FIG. 1 (in which the adhesion rate was determined by regarding the amount of the pharmacopeial barium sulfate adhered per g of the stomach enucleated from the rat in the aqueous solution of pH 1.2 as 100%).

As apparent from FIG. 1, the pharmacopeial barium sulfate exhibited like adhesion rates in the aqueous solutions of the respective pHs. With respect to the polymer-coated barium sulfate preparations, the preparations (Examples 1-3) according to the present invention obtained by coating with a polymer having a carboxyl group exhibited improved adhesiveness under acid conditions (in the aqueous solution of pH 1.2) compared with the pharmacopeial barium sulfate, and exhibited similar adhesion rates to that of the barium sulfate under neutral conditions (in the aqueous solution of pH 7.0) and under alkaline conditions (in the aqueous solution of pH 9.0). On the other hand, the preparation (Comparative Example 1) obtained by coating with a polymer having no carboxyl group exhibited improved adhesiveness in the solutions of the respective pHs compared with the pharmacopeial barium sulfate, but did not show pH-dependent adhesiveness.

### Examples 4-11:

Polymer-coated barium sulfate preparations were obtained in the same manner as in Examples 1-3 except that components of their corresponding formulations shown in Tables 2 and 3 were used.

**Table 2**

| Component | Ex.4 | Ex.5 | Ex.6 | Ex.7 | Ex.8 |
|---|---|---|---|---|---|
| Acrylic acid | 8.65 g | 13.00 g | - | - | - |
| Methacrylic acid | - | - | 15.50 g | 10.33 g | 10.33 g |
| Ethyl acrylate | 17.07 g | - | - | - | - |
| 2-Hydroxypropyl acrylate | - | - | 3.00 g | - | - |
| Ethyl methacrylate | - | - | - | 21.01 g | - |
| n-Butyl methacrylate | - | - | - | - | 6.00 g |
| 2-Hydroxyethyl methacrylate | - | 4.27 g | - | - | - |
| EGDMA | 0.48 g | 0.48 g | 0.48 g | 0.48 g | 0.48 g |
| V-59 | 0.23 g | 0.23 g | 0.23 g | 0.23 g | 0.23 g |
| Pharmacopeial barium sulfate | 52.84 g | 17.98 g | 19.21 g | 23.05 g | 34.08 g |

**Table 3**

| Component | Example 9 | Example 10 | Example 11 |
|---|---|---|---|
| Acrylic acid | 15.50 g | 15.50 g | 15.50 g |
| Methyl methacrylate | 6.00 g | - | - |
| 2-Hydroxyethyl methacrylate | - | 6.00 g | - |
| n-Butyl methacrylate | - | - | 6.00 g |
| EGDMA | 0.24 g | 0.24 g | 0.24 g |
| V-59 | 0.12 g | 0.12 g | 0.12 g |
| Pharmacopeial barium sulfate | 21.86 g | 21.86 g | 21.86 g |

### Test Example 2:

With respect to the respective preparations obtained in Examples 4-11, the adhesiveness to the surface of a mucosa of the stomach enucleated from a rat was investigated. More specifically, each (0.5 g) of the polymer-coated barium sulfate preparations was suspended in each (19.5 g) of aqueous solutions of pH 1.2 and pH 7.0 to determine the amount of the preparation adhered to the surface of the gastric mucosa in the same manner as in Test Example 1. The results are illustrated in FIG. 2 (in which the adhesion rate was determined by regarding the amount of the pharmacopeial barium sulfate adhered per g of the stomach enucleated from the rat in the solution of pH 1.2 as 100%).

As a result, all the preparations exhibited higher adhesion rates under the acid conditions and similar adhesion rates under the neutral conditions compared with the pharmacopeial barium sulfate.

### Examples 12-18:

A methacrylic acid-methyl methacrylate copolymer (Eudragit L100) and triethyl citrate as a plasticizer were dissolved in ethanol in their corresponding amounts shown in Tables 4 and 5, pharmacopeial barium sulfate was suspended in the resultant solutions, and the resultant suspensions were separately sprayed into a drying chamber (temperature in the chamber: 80°C) in which nitrogen gas was circulated, to momentarily evaporate ethanol (spray drying process), thereby obtaining respective polymer-coated barium sulfate preparations. Incidentally, the spray drying device used was Pulvis Mini-Spray GS31 (manufactured by YAMATO SCIENTIFIC CO., LTD.).

**Table 4**

| Component | Ex. 12 | Ex. 13 | Ex. 14 | Ex. 15 |
|---|---|---|---|---|
| Methacrylic acid-methyl methacrylate copolymer (Eudragit L100) | 0.5 g | 1.0g | 2.0 g | 10.0 g |
| Pharmacopeial barium | 100.0 g | 100.0 g | 100.0 g | 100.0 g |
| sulfate | | | | |
| Triethyl citrate | 0.1 g | 0.2 g | 0.4 g | 2.0 g |
| Ethanol | 100 ml | 100 ml | 100 ml | 100 ml |
| Amount of polymer per g of barium sulfate | 0.005 g | 0.01 g | 0.02 g | 0.1 g |

**Table 5**

| Component | Ex. 16 | Ex. 17 | Ex. 18 |
|---|---|---|---|
| Methacrylic acid-methyl methacrylate copolymer (Eudragit L100) | 50.0 g | 100.0 g | 200.0 g |
| Pharmacopeial barium sulfate | 100.0 g | 100.0 g | 100.0 g |
| Triethyl citrate | 10.0 g | 20.0 g | 40.0 g |
| Ethanol | 300 ml | 500 ml | 1000 ml |
| Amount of polymer per g of barium sulfate | 0.5 g | 1.0 g | 2.0 g |

### Test Example 3:

With respect to the preparations of Examples 12-18, in which the amount of the polymer was varied, the adhesiveness to the surface of a mucosa of the stomach enucleated from a rat was investigated. More specifically, each (0.5 g) of the polymer-coated barium sulfate preparations was suspended in an aqueous solution (19.5 g) of pH 1.2 to determine the amount of the preparation adhered to the surface of the gastric mucosa in the same manner as in Test Example 1. The results are illustrated in FIG. 3 (in which the adhesion rate was determined by regarding the amount of the pharmacopeial barium sulfate adhered per g of the stomach enucleated from the rat in the solution of pH 1.2 as 100%).

The preparation (Example 12) in which the amount of the polymer coated was 0.005 g per g of the barium sulfate exhibited an adhesion rate of 124% and was hence somewhat improved in adhesiveness. The adhesion rates of the preparations in which the amount of the polymer were more increased were more enhanced. The preparations in which the amount of the polymer was between 0.02 g and 1 g exhibited an adhesion rate as good as about 300%.

### Test Example 4:

The separation property of the preparation when it was first caused to adhere to the surface of a mucosa of the stomach enucleated from a rat under acid conditions, and then exposed to neutral conditions was investigated. More specifically, the polymer-coated barium sulfate preparation (0.5 g) obtained in Example 16 was suspended in an aqueous solution (19.5 g) of pH 1.2. The stomach enucleated from the rat was placed into the suspension with the surface of the gastric mucosa outside. After the suspension was stirred for 10 minutes, the stomach to which the preparation had adhered was taken out of the suspension. After the stomach was then placed into an aqueous solution (19.5 g) of pH 7.0, and the solution was stirred again for 10 minutes, the stomach was taken out of the solution, thereby determining the amounts of the preparation adhered to the surface of the gastric mucosa at the time the stomach was taken out of the suspension of pH 1.2 and at the time the stomach was taken out of the solution of pH 7.0 by measuring the dry weights of the preparation remaining in both suspension and solution. The results are illustrated in FIG. 4. The amount of the preparation adhered to the surface of the mucosa of the stomach enucleated from the rat was reduced to about a third after the stomach was exposed to the neutral condition through the exposure to the acid conditions.

### INDUSTRIAL APPLICABILITY

The X-ray contrast media according to the present invention have pH-dependent adherability and adhere to the surface of a gastrointestinal mucosa under acid conditions, so that their adhesiveness upon an examination can be enhanced, and their doses and concentrations can be reduced while retaining resolution equivalent to that of the conventional contrast medium. Further, since the contrast media easily separate from the surface of the gastrointestinal mucosa under neutral or alkaline conditions, their excretion can be rapidly conducted by ingesting water and/or food or administering an alkaline substance after the examination, so that side effects such as bellyache, difficulty in defecation and constipation can be relieved.

## Claims

1. An X-ray contrast medium comprising an X-ray contrast substance coated with a polymer which adheres to the surface of a gastrointestinal mucosa under acid conditions and separates from the surface of the gastrointestinal mucosa under neutral or alkaline conditions.

2. The X-ray contrast medium according to Claim 1, wherein the polymer is a polymer exhibiting a pH of 4 or higher when dissolved, and having an anionic group.

3. The X-ray contrast medium according to Claim 2, wherein the anionic group is a carboxyl group.

4. The X-ray contrast medium according to Claim 3, wherein the polymer having a carboxyl group is a polymer obtained from acrylic acid and/or methacrylic acid.

5. The X-ray contrast medium according to Claim 3, wherein the polymer having a carboxyl group is a polymer obtained from acrylic acid and/or methacrylic acid and a carboxylic ester.

6. The X-ray contrast medium according to Claim 5, wherein the carboxylic ester is at least one selected from the group consisting of acrylic esters and methacrylic esters.

7. The X-ray contrast medium according to Claim 5 or 6, wherein the polymer having a carboxyl group is a methacrylic acid-methyl methacrylate copolymer.

8. The X-ray contrast medium according to any one of Claims 1 to 7, wherein the amount of the polymer coated is 0.005-2 g per g of the X-ray contrast substance.

9. The X-ray contrast medium according to any one of Claims 1 to 8, wherein the X-ray contrast substance is barium sulfate.

10. An X-ray contrast composition comprising an X-ray contrast substance coated with a polymer which adheres to the surface of a gastrointestinal mucosa under acid conditions and separates from the surface of the gastrointestinal mucosa under neutral or alkaline conditions, and a pharmaceutically acceptable carrier.

11. Use of an X-ray contrast substance coated with a polymer, which adheres to the surface of a gastrointestinal mucosa under acid conditions and separates from the surface of the gastrointestinal mucosa under neutral or alkaline conditions, for an X-ray contrast medium.

12. A contrast-radiographing process, comprising the steps of administering an X-ray contrast substance coated with a polymer, which adheres to the surface of a gastrointestinal mucosa under acid conditions and separates from the surface of the gastrointestinal mucosa under neutral or alkaline conditions, to a subject and exposing the subject to X-rays.
